# EUROPEAN PATENT SPECIFICATION

(11) **EP 2 290 365 B1**
(45) Date of publication and mention of the grant of the patent: **16.04.2014**
(21) Application number: 09762488.6
(22) Date of filing: 09.06.2009
(51) Int. Cl.: G01N 33/543, G01N 33/553, G01N 35/04

(54) **ANALYZER USING MAGNETIC PARTICLES**
MAGNETISCHE PARTIKEL VERWENDENDER ANALYSATOR
ANALYSEUR UTILISANT DES PARTICULES MAGNÉTIQUES

(30) Priority: 12.06.2008 JP 2008154398
(43) Date of publication of application: 02.03.2011
(73) Proprietor: Hitachi High-Technologies Corporation, Minato-ku Tokyo Tokyo 105-8717 (JP)
(72) Inventor: YAMASHITA Yoshihiro, Hitachinaka-shi Ibaraki 312-8504 (JP); SAKAZUME Taku, Hitachinaka-shi Ibaraki 312-8504 (JP)
(74) Representative: Strehl, Peter
(86) International application number: PCT/JP2009/060548
(87) International publication number: WO 2009/151058

(56) References cited:
- WO-A2-02/37078
- WO-A2-03/049530
- JP-A- 1 136 068
- JP-A- 10 096 732
- JP-A- 2003 012 689
- JP-A- 2006 010 535
- JP-A- 2007 271 411
- JP-T- 2005 522 679
- JP-T- 2007 519 933
- US-A1- 2002 174 878

## Description

### Technical Field

The present invention relates to immunoassay analyzers that use magnetic particles as a solid-phase carrier, and to other analyzers that use magnetic particles, such as a genetic analyzer. More particularly, the invention relates to an analyzer that uses magnetic particles, the analyzer being suitable for separating the magnetic particles from a solution.

### Background Art

Methods for assaying a target substance at high sensitivity by means of immunological specific binding are known as, for example, radioimmunoassay (RIA) that uses radioisotopes as the substances labeled, enzyme immunoassay (EIA) that uses enzyme, electrochemiluminescence assay (ECLIA) that uses chemiluminescent substances. In these analytical methods, to detect an infinitesimal quantity of target substance at high sensitivity, it is common to change the antigens or antibodies having specific affinity into a solid phase, then after binding the target substance to the solid-phase carrier by immunological specific binding, selectively separate the target substance from a liquid phase inclusive of a non-target substance (by means of B/F separation), and isolate and purify the target substance before conducting the detection. During the analyses that involve B/F separation, the separation of solid-phase from liquid-phase employs centrifuging, magnetic separation in which microparticles of a magnetic body or of the glass or resin containing a magnetic body are used as the solid-phase carrier, or other methods. JP 4-45579 B, for example, discloses one such method. In the B/F separation where the microparticles of a substance containing a magnetic body are used as a solid-phase carrier, adopting any one of several methods is considered for improving analytical accuracy.

A first method is executed by accelerating the specific binding of the target substance to the solid-phase carrier, and it is important in the first method to enhance contact efficiency between the target substance in the liquid phase and the surface of the solid-phase carrier by increasing a surface area thereof per weight. In general, magnetic particles of a low specific gravity, which are obtained by chemically plating a resin with a magnetic body, are used, and microparticles from microns to submicrons in particle size are adopted to induce the mobility of the microparticles in a solution due to Brownian motion and improve the dispersibility of the microparticles.

A second method is executed by improving separation/recovery efficiency of the solid-phase carrier from the solution during the B/F separation. In general, the improvement uses a permanent magnet. A magnet and a reaction container are arranged considering the improvement of the strength of a magnetic field working upon the magnetic particles in the solution. The highly dispersible microparticles mentioned above are correspondingly difficult to separate and recover from the solution, and require a long separation/recovery time.

A third method is executed by, prior to the start of analysis, removing any analytical inhibitors that may remain or non-specifically be adsorbed, for example, on the surfaces of the magnetic particles, inside an aggregate thereof, or between the magnetic particles and a reaction container or a magnet. In general, removal of these inhibitors is accomplished by re-dispersing the recovered magnetic particles in such a solution as a washing solution, and eluting or diluting the inhibitors in the solution.

More specifically, the re-dispersion of the magnetic particles in the third method is performed in any one of various ways. For example, JP 10-123136 A discloses stirring the solution containing the magnetic particles, with a stirring tool or by means of air pressure fluctuation, to implement the re-dispersion. JP 2-161358 A proposes fluidizing the solution containing the magnetic particles, by pivoting the reaction container. JP 2006-112824 A describes repeating the re-dispersion and recovery of the magnetic particles by activating alternately a plurality of magnets arranged around the reaction container.

WO 02/37078 A2 discloses an analyzer that has the features set forth in the first part of claim 1.

### Summary of the Invention

The methods described above involve re-dispersing the magnetic particles in the solution, that is, require repeating the recovery of the magnetic particles from the solution. As discussed earlier herein, improving the magnetic particles in dispersibility to improve analytical accuracy during the B/F separation means making the particles correspondingly difficult to separate and recover from the solution. The above conventional methods requiring a long time for the recovery of the magnetic particles, therefore, have posed a problem in that a long time is required for analysis.

An object of the present invention is to provide an analyzer that uses magnetic particles, the analyzer being adapted to remove inhibitors within a short time and thus to minimize the analytical time.

This object is met by the invention defined in claim 1. In an embodiment, an analyzer that uses magnetic particles includes: an incubator disk that retains a reaction container accommodating a reaction solution with the magnetic particles dispersed in the solution, the disk being adapted to heat the reaction container to a predetermined temperature; and a detector unit that, after elapse of a predetermined time of reaction in the reaction container retained by the incubator disk, suctions the reaction solution and detects a target substance bound to the magnetic particles; the analyzer being further inclusive of oscillating means that causes relative positions of a magnet and the reaction container to change in a magnetic field range in which the re-dispersion of the magnetic particles does not occur.

This configuration enables the analyzer to remove inhibitors within a short time and thus to minimize an analytical time.
(2) The oscillating means in above item (1) is preferably vibration-generating means that imparts vibration to the magnetic particles when the particles are in an aggregated condition in the solution.
(3) The analyzer in above item (2) further includes a magnet disposed near a reaction container that accommodates a pre-washing solution with the magnetic particles aggregated in the solution, wherein the vibration-generating means is driving means that repeatedly turns the reaction container.
(4) In item (2), the detector unit includes an electrochemical reaction flow cell through which an electrochemical reaction solution flows with the magnetic particles dispersed therein, and a magnet disposed near the electrochemical reaction flow cell, and the vibration-generating means is driving means that reciprocates the magnet.

### Effects of the Invention

According to the present invention, inhibitors are removed within a short time and thus an analytical time is minimized.

### Brief Description of the Drawings

Fig. 1 is a system block diagram showing an overall configuration of an analyzer using magnetic particles according to an embodiment of the present invention;
Fig. 2 is a system block diagram showing a configuration of a first inhibitor removal mechanism in the analyzer which uses magnetic particles according to the embodiment of the present invention;
Fig. 3 is an operational explanatory diagram of the first inhibitor removal mechanism in the analyzer which uses magnetic particles according to the embodiment of the present invention; and
Fig. 4 is a system block diagram showing a configuration of a second inhibitor removal mechanism in the analyzer which uses magnetic particles according to the embodiment of the present invention.

### Mode for Carrying out the Invention

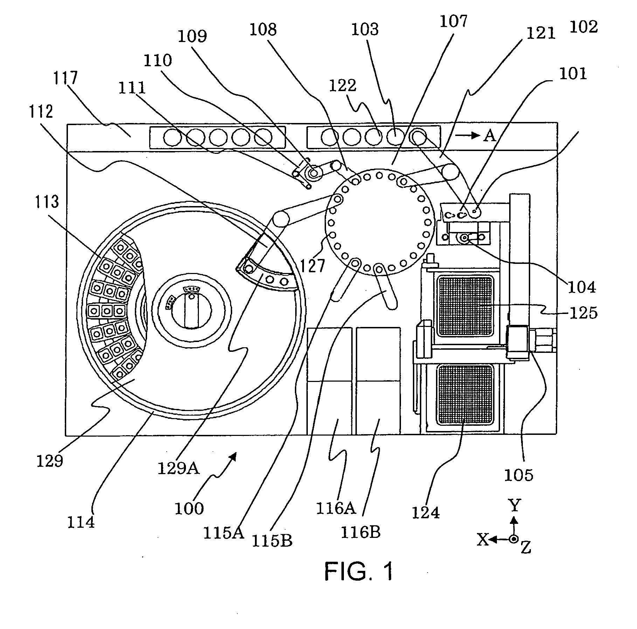

Hereunder, configurations and operation of an analyzer which uses magnetic particles according to an embodiment of the present invention will be described using Figs. 1 to 3.

An overall configuration of the analyzer which uses magnetic particles according to the present embodiment is first described below using Fig. 1. The description here takes an immunoassay analyzer by way of example.

Fig. 1 is a system block diagram showing the overall configuration of the analyzer which uses magnetic particles according to the embodiment of the present invention.

The immunoassay analyzer 100 includes a transport rack 122 in which sample containers 103 each containing a sample are arranged. The transport rack 122 moves in a direction of arrow A, along a sample transport line 117, thus transporting each sample container 103 to a position neighboring a sample-dispensing pipettor 121.

A plurality of reaction containers 127 are placed on an incubator disk 107. The incubator disk 107 is adapted to be rotatable and to move to predetermined respective positions the reaction containers 127 placed in a circumferential direction.

A sample-dispensing tip/reaction container transport mechanism 105 is movable in X-axis, Y-axis, and Z-axis directions. The sample-dispensing tip/reaction container transport mechanism 105 can move within an upper zone that ranges from two sample-dispensing tip/reaction container stations, 124 and 125, to a reaction solution stirring mechanism 104, sample-dispensing tip/reaction container discarding holes 101, a sample-dispensing tip buffer 102, and part of the incubator disk 107, on an X-Y plane. The sample-dispensing tip/reaction container transport mechanism 105 can move vertically in the Z-axis direction, within the above moving zone.

A plurality of unused sample-dispensing tips and a plurality of unused reaction containers are placed in the sample-dispensing tip/reaction container stations 124 and 125, respectively. The sample-dispensing tip/reaction container transport mechanism 105 moves above the sample-dispensing tip/reaction container stations 124 and 125 in a range of the X-Y plane, then after descending in the Z-axis direction, grips one of the unused reaction containers, and ascends once again. Next after thus further moving above the incubator disk 107 in the X-Y plane range, the sample-dispensing tip/reaction container transport mechanism 105 descends Z-axially once again and places the reaction container at a reaction container mounting position on the incubator disk 107.

The sample-dispensing tip/reaction container transport mechanism 105 also moves above the sample-dispensing tip/reaction container stations 124 and 125 in the X-Y plane range, then after descending Z-axially, grips one of the unused sample-dispensing tips, and ascends once again. Next after thus further moving above the sample-dispensing tip buffer 102 in the X-Y plane range, the sample-dispensing tip/reaction container transport mechanism 105 descends Z-axially once again and places the sample-dispensing tip in the sample-dispensing tip buffer 102.

The sample-dispensing pipettor 121 is adapted to turn and to move vertically. The sample-dispensing pipettor 121 rotationally moves above the sample-dispensing tip buffer 102 and then moves downward to mount the sample-dispensing tip at a distal end of the pipettor. The sample-dispensing pipettor 121 on which the sample-dispensing tip has been mounted moves above one of the sample containers 103 rested in the transport rack 122, then moves downward, and suctions a predetermined amount of sample retained by the sample container 103. Upon completion of suctioning the sample, the sample-dispensing pipettor 121 moves above the incubator disk 107, and after moving downward, dispenses the sample into the unused reaction container 127 retained on the incubator disk 107. When analyses over a plurality of items are to be performed upon one sample, the sample-dispensing pipettor 121 uses one sample-dispensing tip to repeatedly suction the sample retained by one sample container 103 and dispense the sample into another unused reaction container retained by the incubator disk 107. Upon completion of dispensing the sample, the sample-dispensing pipettor 121 moves above one of the sample-dispensing tip/reaction container discarding holes 101 and discards the used sample-dispensing tip from the discarding hole.

Reagents retained in a plurality of reagent containers are placed on a reagent disk 114. The reagent disk 114 has a cover or lid 129 on an upper section of the disk, and the reagent disk 114 includes a cold storage space formed inside thereof. An opening 129A is provided in a part of the cover or lid 129. A reagent-dispensing pipettor 112 is constructed to turn and to move vertically. The reagent-dispensing pipettor 112 moves above the opening 129A in the cover or lid 129 of the reagent disk 114, then suctions a predetermined kind of reagent in a predetermined amount, and after moving above the incubator disk 107, dispenses the reagent into a reaction container 127 containing the dispensed sample.

The reaction container 127 containing the dispensed sample and reagent then moves to a position within the X-Y planar moving zone of the sample-dispensing tip/reaction container transport mechanism 105. The sample-dispensing tip/reaction container transport mechanism 105 descends Z-axially and after gripping the reaction container 127 with the sample and reagent dispensed thereinto, moves to set up the reaction container 127 at the reaction solution stirring mechanism 104. The reaction solution stirring mechanism 104 stirs the sample and reagent within the reaction container by imparting rotary motion thereto. The reaction container in which the stirring operation has been completed is returned to the incubator disk 107 by the sample-dispensing tip/reaction container transport mechanism 105. The inside of the incubator disk 107 is maintained at a constant temperature.

The incubator disk 107 has two reaction solution suction nozzles, 115A and 115B, on its outer surface. After elapse of a predetermined time of reaction on the incubator disk 107 following the stirring of the sample and the reagent, the reaction solution in the reaction container is suctioned by the reaction solution suction nozzles 115A, 115B. The reaction solution that has been suctioned from the reaction container by the reaction solution suction nozzle 115A is detected by a detector 116A. The reaction solution that has likewise been suctioned from the reaction container by the reaction solution suction nozzle 115B is detected by a detector 116B. The detectors 116A and 116B are of the same configuration, and the two units are equipped for obtaining higher throughput by reducing a detection time.

The reaction container from which the reaction solution has been suctioned moves to a position within the moving zone of the sample-dispensing tip/reaction container transport mechanism 105 by the turning action of the incubator disk 107. The sample-dispensing tip/reaction container transport mechanism 105 removes the used reaction container from the incubator disk 107 and discards the reaction container into one reaction container discarding hole of the sample-dispensing tip/reaction container discarding holes 101.

A pre-washing station 109 is used only for predetermined analytical items. Some specific properties of analytes are significantly affected by inhibitors, therefore requiring prior removal of inhibitors as many as possible from the analyte. The pre-washing station 109 is used for such analytical items. After the elapse of the predetermined time of reaction on the incubator disk 107 following the stirring of the sample and the reagent, the reaction container is moved to the pres-washing station 109 by a pre-washing transport mechanism 108. A pre-washing solution delivery nozzle 110 and a reaction solution suction nozzle 111 for a pre-washing solution are provided near the pre-washing station 109.

When a magnet is brought close to the reaction container, the magnetic particles in the reaction solution are aggregated and then retained on an inner wall of the container. Under this state, the reaction solution suction nozzle 111 for a pre-washing solution suctions the reaction solution from the reaction container that has been moved to the pres-washing station 109. During the suctioning of the reaction solution, inhibitors are also suctioned. This results in the magnetic particles (beads) remaining in the reaction container. After this, the pre-washing solution delivery nozzle 110 delivers a pre-washing solution into the reaction container. A buffer solution, for example, is used as the pre-washing solution. After pre-washing, the magnet leaves the reaction container. Next after the magnetic particles and pre-washing solution in the reaction container have been redispersed by stirring, the pre-washing transport mechanism 108 returns the reaction container to the incubator disk 107. After this, the liquid (a mixture of the magnetic particles and the pre-washing solution) in the reaction container is suctioned by the reaction solution suction nozzles 115A, 115B, and then detected by the detectors 116A, 116B.

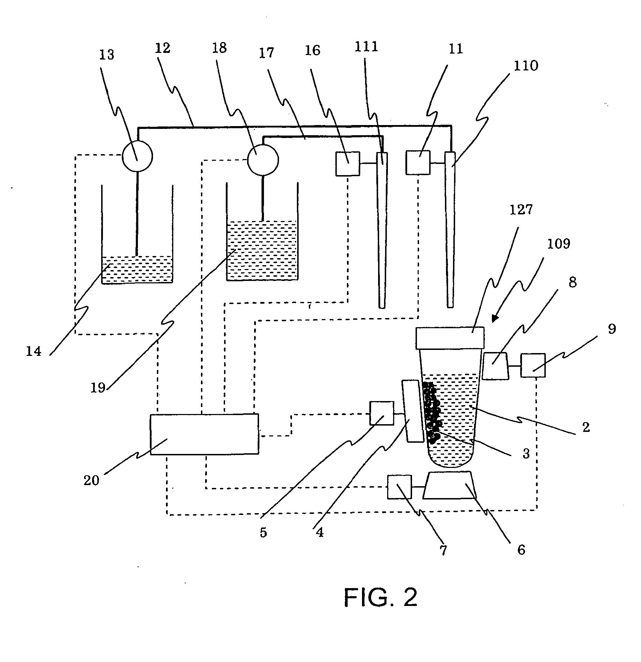

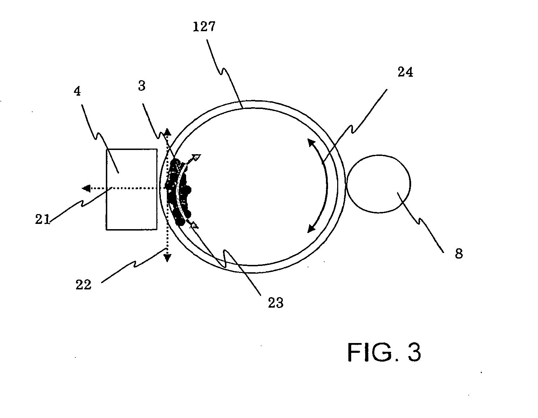

Next, a first inhibitor removal mechanism in the analyzer which uses magnetic particles according to the present embodiment is described below using Figs. 2 and 3. Fig. 2 is a system block diagram showing a configuration of the first inhibitor removal mechanism in the analyzer which uses magnetic particles according to the embodiment of the present invention. Fig. 3 is an operational explanatory diagram of the first inhibitor removal mechanism in the analyzer which uses magnetic particles according to the embodiment of the present invention. Referring to Figs. 2 and 3, the same reference numbers as those used in Fig. 1 denote the same constituent elements.

The first inhibitor removal mechanism is provided at the pre-washing station 109 shown in Fig. 1.

As described in Fig. 1, each reaction container 127 is moved to the pre-washing station 109 by the pre-washing transport mechanism 108. A solution 2 within the reaction container 127 contains magnetic particles 3 existing in a dispersed condition. The solution 2 contains a substance to be assayed (an analyte), antibodies each for specifically binding the analyte and one magnetic particle together, and the like. That is to say, in a reaction solution, the analyte and the magnetic particle form a complex via the corresponding antibody or the like. Unreacted substances not forming a complex can be inhibitors with respect to subsequent reactions. Therefore, B/F separating operations are performed to isolate the complex of the analyte and the magnetic particle from other (unreacted) substances and purify the isolated complex.

A control unit 20 controls a magnet-driving motor 5 to bring a magnet 4 into close proximity to the reaction container 127, thereby causing the magnetic particles 3 in the reaction solution to be magnetically recovered on the inner wall of the container 127 to which the magnet is in close proximity.

Under this state, a solution suction nozzle driving motor 16 positions the reaction solution suction nozzle 111 centrally above the reaction container 127 and next moves a distal end of the reaction solution suction nozzle 111 downward to a position adjacent to an inner bottom section of the container. After this, the reaction solution is suctioned by a solution suction pump 18 and then pumped into a suctioned-solution tank 19 through a solution suction line 17. Thus, only the magnetic particles 3 remain in the reaction container 127. The solution suction nozzle driving motor 16 next returns the reaction solution suction nozzle 111 to an initial position thereof.

Next, a solution supply nozzle driving motor 11 positions the pre-washing solution delivery nozzle 110 centrally above the reaction container 127 and then moves a distal end of the pre-washing solution delivery nozzle 110 downward to a position adjacent to the inner bottom section of the container. Next, a solution supply pump 13 pumps up the pre-washing solution from a pre-washing solution supply tank 14 and then pumps the pre-washing solution out from the pre-washing solution delivery nozzle through a solution supply line 12 into the reaction container. This pre-washing solution is a buffer solution or the like, acting as a washing solution. The washing solution can be a diluent or eluent for the inhibitors left inside an aggregate of the magnetic particles, on the surfaces of the magnetic particles, and on the inner wall of the container. After the delivery of the pre-washing solution, the solution supply nozzle driving motor 11 returns the pre-washing solution delivery nozzle 110 to an initial position thereof.

Next, a container-rotating gear driving motor 9, by repetitively rotating a container-rotating gear 8 that comes into firm contact with an outer wall of the reaction container 127, repetitively rotates the reaction container 127 in container rotational directions 24. This, as shown in Fig. 3, iteratively moves the magnetic particles 3 in moving directions 23 thereof by an action of a magnetic force applied in a direction 21, and an action of a frictional force exerted in a frictional direction 22. Magnetic field strength that the magnet 4 gives to the magnetic particles 3 at this time stays in a magnetic field strength range in which the magnetic particles 3 do not become redispersed in the pre-washing solution. Therefore, the magnetic particles 3 each change in relative position on the inner wall of the container without redispersing. The magnetic particles 3 are spherical. Slight clearances exist between the magnetic particles densely populated by the magnetic fields, and sticking inhibitors are present at the clearances. Under this state, changing the relative positions of the magnetic particles 3 on the inner wall of the container, that is, vibrating the magnetic particles 3 changes the relationship in position between the magnetic particles 3 densely populated by the magnetic fields, and thus changes the clearances between the magnetic particles. This, in turn, causes any inhibitors included in an aggregate of the magnetic particles, any inhibitors remaining between the magnetic particles and the container or the magnet, and any inhibitors nonspecifically adsorbed on the magnetic particles, to be efficiently eluted or diluted in the washing solution. Briefly, the motor 9 that repetitively rotates the reaction container acts as a vibrator for the magnetic particle aggregate and can therefore remove unnecessary inhibitors from the magnetic particles. After inhibitor removal, the container-rotating gear stops the repetitive rotation, thus stopping the repetitive rotation of the container.

After this, the solution suction pump 18 suctions the pre-washing solution and pumps the pre-washing solution into the suctioned-solution tank 19 through solution suction line 17. The solution suction nozzle driving motor 16 next returns the reaction solution suction nozzle 111 to the initial position.

Next, the solution supply nozzle driving motor 11 positions the pre-washing solution delivery nozzle 110 centrally above the reaction container 127 and then moves the distal end of the pre-washing solution delivery nozzle 110 downward to a position adjacent to the inner bottom section of the container. The solution supply pump 13 then pumps up the pre-washing solution from the pre-washing solution supply tank 14 and pumps the pre-washing solution out from the pre-washing solution delivery nozzle 110 through the solution supply line 12 into the reaction container.

The repetitive rotation of the reaction container and the suctioning and supply of the pre-washing solution can be repeated according to particular needs.

After that, the control unit 20 controls a stirring-table driving motor 7 to drive a stirring table 6. The stirring table 6 rotates a lower section of the reaction container 127 in a circumferential direction while the container is being retained at its upper section. Thus, the reaction container 127 is given an eccentric circular motion, which then disperses the magnetic particles existing inside the reaction container.

As means for changing the relative positions of the magnetic particles 3 aggregated onto the inner wall of the reaction container 127 by the magnetic fields, using the magnet-driving motor 5 may be considered to repeatedly move the magnet 4 upward and downward or turn the magnet, along the outer wall of the reaction container 127, while maintaining the magnet 4 in close proximity to the container. Alternatively, the reaction container 127 may be gripped by means of the pre-washing transport mechanism 108 and repeatedly be moved upward and downward or turned while the magnet 4 is maintaining its close proximity to the reaction container 127.

Highly dispersible magnetic particles are difficult to separate and recover from the solution. Conventional technology for removing inhibitors by repeating the dispersion/recovery of magnetic particles, therefore, requires a long time for the re-recovery of the magnetic particles. For this reason, the time required for the removal of inhibitors in the conventional technology is nearly 30 seconds, for example. In the present embodiment, however, a time requirement for the removal of inhibitors can be reduced to, for example, nearly 10 seconds, since during the suctioning of the reaction solution, the delivery of the pre-washing solution, the suctioning of the pre-washing solution, and ensuing delivery of the pre-washing solution, the magnetic particles remain aggregated by the magnet, and since the aggregated magnetic particles are neither redispersed nor re-recovered. Yet, imparting a vibration to the aggregated magnetic particles by repeatedly rotating the reaction container is likewise an effective method for removing any inhibitors remaining at the clearances between the aggregated magnetic particles.

As described, according to the present embodiment, during the B/F separation that uses magnetic particles as a solid-phase carrier, inhibitors contained in an aggregate of the magnetic particles, inhibitors remaining between the magnetic particles and the corresponding container or a magnet, and inhibitors that nonspecifically adsorbed onto the magnetic particles are removed effectively without redispersing/re-recovering the magnetic particles. Improvement of analytical accuracy and reduction in analytical time are therefore implemented.

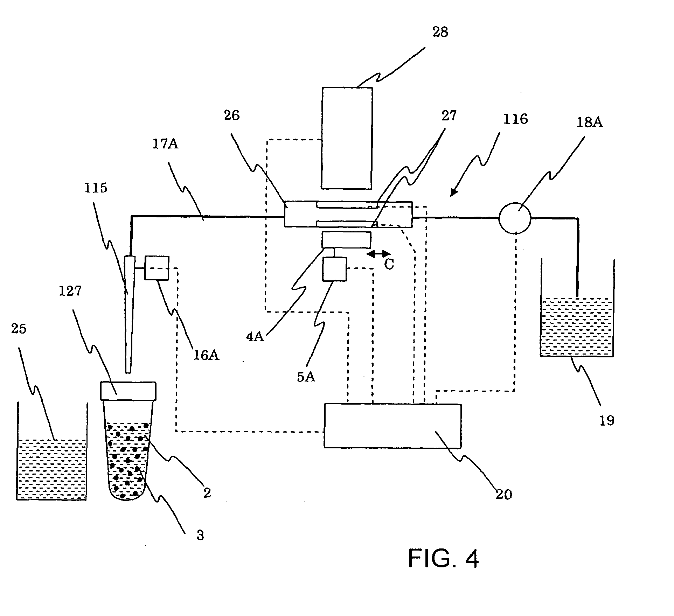

Next, a second inhibitor removal mechanism in the analyzer which uses magnetic particles according to the present embodiment is described below using Fig. 4. Fig. 4 is a system block diagram showing a configuration of the second inhibitor removal mechanism in the analyzer which uses magnetic particles according to the embodiment of the present invention. Referring to Fig. 4, the same reference numbers as those used in Fig. 1 denote the same constituent elements.

The second inhibitor removal mechanism is provided at the detector unit 116 shown in Fig. 1. The detector unit 116 includes an electrochemiluminescence detection flow cell. Electrochemiluminescence is a phenomenon in which an electrochemiluminescent substance such as a metal chelate will emit light when excited by electrode reactions. The electrochemiluminescence is utilized to detect microsubstances such as antigens and antibodies.

In a reaction container 127 retained by the incubator disk 107 shown in Fig. 1, upon a predetermined reaction time elapsing, a solution 2 contains the magnetic particles 3 in a dispersed state. The solution 2 also contains an analyte, antibodies each for binding the analyte and each dispersed magnetic particle together, and the like. That is to say, in a reaction solution, the analyte and the magnetic particle form a complex via the corresponding antibody or the like. Unreacted substances not forming a complex, however, can be inhibitors with respect to subsequent reactions. Therefore, B/F separating operations are performed to isolate the complex of the analyte and the magnetic particle from other (unreacted) substances and purify the isolated complex. First, the control unit 20 controls a solution suction nozzle driving motor 16A to position a reaction solution suction nozzle 115 centrally above the reaction container 127 and next move a distal end of the reaction solution suction nozzle 115 downward to a position adjacent to an inner bottom section of the container. After this, the reaction solution is suctioned by a solution suction pump 18A and then the suctioned reaction solution is pumped into the electrochemical reaction flow cell 26 through a solution suction line 17A.

Next, the control unit 20 controls a magnet-driving motor 5 to bring a magnet 4 into close proximity to a lower face of the electrochemical reaction flow cell 26, and causes the internal magnetic particles of the reaction solution that pass through the electrochemical reaction flow cell 26, to be magnetically adsorbed onto the surface of a lower reaction electrode of paired reaction electrodes 27.

Next, the control unit 20 activates the solution suction nozzle driving motor 16A to immerse the reaction solution suction nozzle 115 in the electrochemical reaction solution 25, and operates the solution suction pump 18A to make the suction nozzle 115 suction the reaction solution and then pump the suctioned reaction solution into the electrochemical reaction flow cell 26 through the solution suction line 17A. At this time, the control unit 20 uses the magnet-driving motor 5A to repeatedly move the magnet 4A in parallel directions (directions of arrow C in Fig. 4) to the fluid while maintaining the magnet 4A in close proximity to the lower face of the electrochemical reaction flow cell. This makes the magnetic particles change the respective relative positions on the surface of the reaction electrode within the electrochemical reaction flow cell, in the magnetic field range where the magnetic particles do not become redispersed. Magnetic field strength that the magnet 4A gives to the magnetic particles at this time lies in the magnetic field strength range in which the magnetic particles do not become redispersed. Therefore, the magnetic particles each change in relative position on the inner wall of the container without redispersing. In other words, vibrating the magnetic particles changes the relative positions of the particles densely populated by the magnetic fields, and thus changes the clearances between the magnetic particles. This, in turn, causes any inhibitors included in an aggregate of the magnetic particles, any inhibitors remaining between the magnetic particles and the container or the magnet, and any inhibitors nonspecifically adsorbed on the magnetic particles, to be efficiently eluted or diluted in the washing solution. Briefly, the motor 5A that repetitively rotates the magnet 4A acts as a vibrator for the magnetic particle aggregate and can therefore remove unnecessary inhibitors from the magnetic particles.

After inhibitor removal, the control unit 20 stops the operation of the solution suction pump 18A, thus immobilizing the solution, and activating the magnet-driving motor 5A to move the magnet 5A away from the lower face of the electrochemical reaction flow cell 26.

Next, the control unit 20 applies voltage to the reaction electrodes 27 and induces an electrochemiluminescent reaction. The amount of light emitted from this reaction will be measured by a luminescence detector 28 mounted outside the electrochemical reaction flow cell 26.

As described, according to the present embodiment, during the B/F separation that uses magnetic particles as a solid-phase carrier, inhibitors contained in an aggregate of the magnetic particles, inhibitors remaining between the magnetic particles and the corresponding container or a magnet, and inhibitors that nonspecifically adsorb onto the magnetic particles are removed effectively without redispersing/re-recovering the magnetic particles. Improvement of analytical accuracy and reduction in analytical time are therefore implemented.

### Description of Reference Numerals

- 2: Solution
- 3: Magnetic particle
- 4, 4A: Magnets
- 5, 5A: Magnet-driving motor
- 6: Stirring table
- 7: Stirring-table driving motor
- 8: Container-rotating gear
- 9: Container-rotating gear driving motor
- 10: Solution supply nozzle
- 11: Solution supply nozzle driving motor
- 12: Solution supply line
- 13: Solution supply pump
- 14: Pre-washing solution tank
- 15: Solution suction nozzle
- 16, 16A: Solution suction nozzle driving motors
- 17, 17A: Solution suction lines
- 18, 18A: Solution suction pumps
- 19, 19A: Suctioned-solution tanks
- 20: Control unit
- 25: Electrochemical reaction solution
- 26: Electrochemical reaction flow cell
- 27: Reaction electrode
- 28: Luminescence detector
- 100: Immunoassay analyzer
- 103: Sample container
- 105: Sample-dispensing tip/reaction container transport mechanism
- 107: Incubator disk
- 108: Pre-washing transport mechanism
- 109: Pre-washing station
- 112: Reagent-dispensing pipettor
- 114: Reagent disk
- 115: Reaction solution suction nozzle
- 116: Detector unit
- 121: Sample-dispensing pipettor
- 124: Sample-dispensing tip station
- 125: Reaction container station
- 127: Reaction container

## Claims

1. An analyzer using magnetic particles, comprising:
a container (26) for accommodating a reaction solution with the magnetic particles, to which a target substance to be analysed is bound, dispersed therein;
a magnet (4, 4A) for aggregating the magnetic particles present in the container (26);
means (110, 115) for supplying a solution for separating inhibitors from the magnetic particles; and
means for maintaining the magnetic field exerted by the magnet (4) within a range in which the magnetic particles once aggregated do not become re-dispersed,
**characterized by** means (5) for changing the relative position between the magnet (4) and the container (26) to cause the relative positions between the magnetic particles to change while the separating solution is being supplied.

2. The analyzer of claim 1, including vibration-generating means (8) for imparting vibration to the magnetic particles while they are in an aggregated condition in the solution.

3. The analyzer of claim 2, wherein the magnet is disposed near the container (26) and the vibration-generating means (8) is adapted to repetitively turn the container (26).

## Patentansprüche

1. Mit magnetischen Partikeln arbeitender Analysator mit:
einem Behälter (26) zur Aufnahme einer Reaktionslösung, in der die magnetischen Teilchen, an die eine zu analysierende Zielsubstanz gebunden ist, dispergiert ist,
einem Magnet (4, 4A) zum Sammeln der in dem Behälter (26) vorhandenen magnetischen Teilchen,
einer Einrichtung (110, 115) zum Zuführen einer Lösung zum Trennen von Inhibitoren von den magnetischen Teilchen und
einer Einrichtung zum Aufrechterhalten des von dem Magnet (4) ausgeübten Magnetfeldes in einem Bereich, in dem die magnetischen Teilchen nach ihrer Aggregation nicht wieder dispergieren,
**gekennzeichnet durch** eine Einrichtung (5) zum Ändern der relativen Position zwischen dem Magnet (4) und dem Behälter (26) derart, dass sich die relativen Positionen zwischen den magnetischen Teilchen während der Zuführung der Trennlösung ändern.

2. Analysator nach Anspruch 1 mit einer Vibrationseinrichtung (8) zum Beaufschlagen der magnetischen Teilchen mit Vibrationen, während sie sich in einem aggregierten Zustand in der Lösung befinden.

3. Analysator nach Anspruch 2, wobei der Magnet nahe dem Behälter (26) angeordnet und die Vibrationseinrichtung (8) so ausgelegt ist, dass sie den Behälter (26) wiederholt dreht.

## Revendications

1. Analyseur qui utilise des particules magnétiques, comportant :
un récipient (26) pour recevoir une solution de réaction avec les particules magnétiques, auxquelles une substance cible à analyser est liée, dispersées dans celle-ci,
un aimant (4, 4A) pour agréger les particules magnétiques présentes dans le récipient (26),
des moyens (110, 115) pour fournir une solution afin de séparer des inhibiteur des particules magnétiques, et
des moyens pour maintenir le champ magnétique exercé par l'aimant (4) à une portée dans laquelle les particules magnétiques une fois agrégées ne se dispersent pas à nouveau,
**caractérisé par** des moyens (5) pour changer la position relative entre l'aimant (4) et le récipient (26) afin d'amener les positions relatives entre les particules magnétiques à changer alors que la solution de séparation est en cours de délivrance.

2. Analyseur selon la revendication 1, incluant des moyens de génération de vibration (8) pour communiquer une vibration aux particules magnétiques alors qu'elles sont dans un état agrégé dans la solution.

3. Analyseur selon la revendication 2, dans lequel l'aimant est disposé à proximité du récipient (26) et les moyens de génération de vibration (8) sont adaptés pour tourner le récipient (26) de manière répétitive.
